# EUROPÄISCHE PATENTSCHRIFT

(11) **EP 1 228 246 B1**
(45) Veröffentlichungstag und Bekanntmachung des Hinweises auf die Patenterteilung: **20.07.2005**
(21) Anmeldenummer: 00987010.6
(22) Anmeldetag: 13.10.2000
(51) Int. Cl.: C12Q 1/68

(54) **VERFAHREN ZUR UNTERSCHEIDUNG VON 5-POSITION METHYLIERUNGSÄNDERUNGEN**
METHOD FOR DISTINGUISHING 5-POSITION METHYLATION CHANGES
PROCEDE PERMETTANT DE DISTINGUER LES MODIFICATIONS DE METHYLATION EN POSITION 5

(30) Priorität: 15.10.1999 DE 19951189
(43) Veröffentlichungstag der Anmeldung: 07.08.2002
(73) Patentinhaber: Epigenomics AG, 10178 Berlin (DE)
(72) Erfinder: BERLIN, Kurt, 14532 Stahnsdorf (DE)
(74) Vertreter: Schubert, Klemens, Dr.
(86) Internationale Anmeldenummer: PCT/DE2000/003726
(87) Internationale Veröffentlichungsnummer: WO 2001/027317

(56) Entgegenhaltungen:
- WO-A-99/10540
- WO-A-99/28498
- US-A- 5 786 146
- WARNECKE P M ET AL: "Detection and measurement of PCR bias in quantitative methylation analysis of bisulphite-treated DNA" NUCLEIC ACIDS RESEARCH,GB,OXFORD UNIVERSITY PRESS, SURREY, Bd. 25, Nr. 21, 1997, Seiten 4422-4426, XP002090424 ISSN: 0305-1048
- XIONG Z AND LAIRD P W: "COBRA: a sensitive and quantitative DNA methylation assay" NUCLEIC ACIDS RESEARCH,GB,OXFORD UNIVERSITY PRESS, SURREY, Bd. 25, Nr. 12, 1997, Seiten 2532-2534, XP002106407 ISSN: 0305-1048
- GONZALGO M L AND JONES P A: "Rapid quantitation of methylation differences at specific sites using methylation-sensitive single nucleotide primer extension (Ms-SNuPE)" NUCLEIC ACIDS RESEARCH,GB,OXFORD UNIVERSITY PRESS, SURREY, Bd. 25, Nr. 12, 15. Juni 1997 (1997-06-15), Seiten 2529-2531, XP002089928 ISSN: 0305-1048
- HERMAN J G ET AL: "METHYLATION-SPECIFIC PCR: A NOVEL PCR ASSAY FOR METHYLATION STATUS OF CPG ISLANDS" PROCEEDINGS OF THE NATIONAL ACADEMY OF SCIENCES OF USA,US,NATIONAL ACADEMY OF SCIENCE. WASHINGTON, Bd. 93, 1. September 1996 (1996-09-01), Seiten 9821-9826, XP002910406 ISSN: 0027-8424

## Beschreibung

Die vorliegende Erfindung betrifft ein Verfahren zur Unterscheidung von 5-Position Methylierungsänderungen von Cytosin-Basen und Cytosin-zu-Thymin Mutationen und zum Nachweis von single nucleotide polymorphisms (SNPs) oder Punktmutationen in genomischer DNA

Die nach den methodischen Entwicklungen der letzten Jahre in der Molekularbiologie gut studierten Beobachtungsebenen sind die Gene selbst, die Übersetzung dieser Gene in RNA und die daraus entstehenden Proteine. Wann im Laufe der Entwicklung eines Individuums welches Gen angeschaltet wird und wie Aktivieren und Inhibieren bestimmter Gene in bestimmten Zellen und Geweben gesteuert wird, ist mit hoher Wahrscheinlichkeit mit Ausmaß und Charakter der Methylierung der Gene bzw. des Genoms korrelierbar. Insofern ist die Annahme naheliegend, daß pathogene Zustände sich in einem veränderten Methylierungsmuster einzelner Gene oder des Genoms äußern.

Die vorliegende Erfindung beschreibt ein Verfahren zur Detektion des Methylierungszustandes genomischer DNA Proben, wobei insbesondere Mutationen und Cytosin Methylierungen voneinander unterschieden werden können. Das Verfahren kann auch zum Auffinden von Punktmutationen und Single Nucleotide Polymorphisms (SNPs) genutzt werden.

5-Methylcytosin ist die häufigste kovalent modifizierte Base in der DNA eukaryotischer Zellen. Sie spielt beispielsweise eine Rolle in der Regulation der Transkription, genomischem Imprinting und in der Tumorgenese. Die Identifizierung von 5-Methylcytosin als Bestandteil genetischer Information ist daher von erheblichem Interesse. 5-Methylcytosin-Positionen können jedoch nicht durch Sequenzierung identifiziert werden, da 5-Methylcytosin das gleiche Basenpaarungsverhalten aufweist wie Cytosin. Darüber hinaus geht bei einer PCR-Amplifikation die epigenetische Information, welche die 5-Methylcytosine tragen, vollständig verloren.

Die Modifikation der genomischen Base Cytosin zu 5'-Methylcytosin stellt den bis heute wichtigsten und bestuntersuchten epigenetischen Parameter dar. Trotzdem gibt es bis heute zwar Methoden, umfassende Genotypen von Zellen und Individuen zu ermitteln, aber noch keine vergleichbaren Ansätze auch in großem Maße epigenotypische Information zu generieren und auszuwerten.

In WO 99/10540 wird eine Methode zur Verwendung einer DNA-Methyltransferase für die Genotypisierung beschrieben. Hierbei werden CG-Stellen aufmethyliert und diese aufmethylierten Stellen anhand einer immunchemischen oder radioaktiven Markierung nachgewiesen. Damit werden Mutationen oder Polymorphismen nachgewiesen. Der Nachteil dieses Verfahrens besteht darin, dass nicht gleichzeitig 5-Position Methylierungsänderungen, Cytosin-zu-Thymin Mutationen, single nucleotide polymorphism (SNPs) oder Punktmutationen in genomischer DNA nachgewiesen werden können.

Es sind im Prinzip drei prinzipiell verschiedene Methoden bekannt, den 5-Methyl-Status eines Cytosins im Sequenzkontext zu bestimmen.

Die erste prinzipielle Methode beruht auf der Verwendung von Restriktionsendonukleasen (RE), welche "methylierungssensitiv" sind. REs zeichnen sich dadurch aus, daß sie an einer bestimmten DNA-Sequenz, meist zwischen 4 und 8 Basen lang, einen Schnitt in die DNA einführen. Die Position solcher Schnitte kann dann durch Gelektrophorese, Transfer auf eine Membran und Hybridisierung nachgewiesen werden. Methylierungssensitiv bedeutet, daß bestimmte Basen innerhalb der Erkennungssequenz unmethyliert vorliegen müssen, damit der Schnitt erfolgen kann. Das Bandenmuster nach einem Restriktionsschnitt und Gelektrophorese ändert sich also je nach Methylierungsmuster der DNA. Allerdings befinden sich die wenigsten methylierbaren CpG innerhalb von Erkennungssequenzen von REs, können also nach dieser Methode nicht untersucht werden.

Die Empfindlichkeit dieser Methoden ist extrem niedrig (Bird, A.P., and Southern, E.M., J.Mol. Biol. 118, 27-47). Eine Variante kombiniert PCR mit dieser Methode, eine Amplifikation durch zwei auf beiden Seiten der Erkennungssequenz liegende Primer erfolgt nach einem Schnitt nur dann, wenn die Erkennungssequenz methyliert vorliegt. Die Empfindlichkeit steigt in diesem Fall auf theoretisch ein einziges Molekül der Zielsequenz, allerdings können mit hohem Aufwand nur einzelne Positionen untersucht werden (Shemer, R. et al., PNAS 93, 6371-6376). Wiederum ist Voraussetzung, daß sich die methylierbare Position innerhalb der Erkennungssequenz einer RE befindet.

Die zweite Variante beruht auf partieller chemischer Spaltung von Gesamt-DNA, nach dem Vorbild einer Maxam-Gilbert Sequenzierreaktion, Ligation von Adaptoren an die so generierten Enden, Amplifikation mit generischen Primern und Auftrennung auf einer Gelektrophorese. Mit diesem Verfahren können definierte Bereiche bis zur Größe von weniger als tausend Basenpaaren untersucht werden. Das Verfahren ist allerdings so kompliziert und unzuverlässig, daß es praktisch nicht mehr verwendet wird (Ward, C. et al., J. Biol. Chem. 265, 3030-3033).

Eine relativ neue und die mittlerweile am häufigsten angewandte Methode zur Untersuchung von DNA auf 5-Methylcytosin beruht auf der spezifischen Reaktion von Bisulphit mit Cytosin, das nach anschließender alkalischer Hydrolyse in Uracil umgewandelt wird, welches in seinem Basen-Paarungsverhalten dem Thymidin entspricht. 5-Methylcytosin wird dagegen unter diesen Bedingungen nicht modifiziert. Damit wird die ursprüngliche DNA so umgewandelt, daß Methylcytosin, welches ursprünglich durch sein Hybridisierungsverhalten vom Cytosin nicht unterschieden werden kann, jetzt durch "normale" molekularbiologische Techniken als einzig verbliebenes Cytosin beispielsweise durch Amplifikation und Hybridisierung oder Sequenzierung nachgewiesen werden kann. Alle diese Techniken beruhen auf Basenpaarung, welche jetzt voll ausgenutzt werden kann. Der Stand der Technik, was die Empfindlichkeit betrifft, wird durch ein Verfahren definiert, welches die zu untersuchende DNA in einer Agarose-Matrix einschließt, dadurch die Diffusion und Renaturierung der DNA (Bisulphit reagiert nur an einzelsträngiger DNA) verhindert und alle Fällungs- und Reinigungsschritte durch schnelle Dialyse ersetzt (Olek, A. et al., Nucl. Acids. Res. 24, 5064-5066). Mit dieser Methode können einzelne Zellen untersucht werden, was das Potential der Methode veranschaulicht. Allerdings werden bisher nur einzelne Regionen bis etwa 3000 Basenpaare Länge untersucht, eine globale Untersuchung von Zellen auf Tausende von möglichen Methylierungsereignissen ist nicht möglich. Allerdings kann auch dieses Verfahren keine sehr kleinen Fragmente aus geringen Probenmengen zuverlässig analysieren. Diese gehen trotz Diffusionsschutz durch die Matrix verloren.

Eine Übersicht über die weiteren bekannte Möglichkeiten, 5-Methylcytsosine nachzuweisen, kann auch dem folgenden Übersichtsartikel entnommen werden: Rein, T., DePamphilis, M. L., Zorbas, H., Nucleic Acids Res. 26, 2255 (1998).

Die Bisulphit-Technik wird bisher bis auf wenige Ausnahmen (z.B. Zeschnigk, M. et al., Eur. J. Hum. Gen. 5, 94-98; Kubota T. et al., Nat. Genet. 16, 16-17) nur in der Forschung angewendet. Immer aber werden kurze, spezifische Stücke eines bekannten Gens nach einer Bisulphit-Behandlung amplifiziert und entweder komplett sequenziert (Olek, A. and Walter, J., Nat. Genet. 17, 275-276) oder einzelne Cytosin-Positionen durch eine "Primer-Extension-Reaktion" (Gonzalgo, M. L. and Jones, P.A., Nucl. Acids. Res. 25, 2529-2531) oder Enzymschnitt (Xiong, Z. and Laird, P.W., Nucl. Acids. Res. 25, 2532-2534) nachgewiesen. Zudem ist auch der Nachweis durch Hybridisierung beschrieben worden (Olek et al., WO99 28498).

Weitere Publikationen, die sich mit der Anwendung der Bisulfit-Technik zum Methylierungsnachweis bei einzelnen Genen befassen, sind: Xiong, Z. and Laird, P.W. (1997), Nucl. Acids Res. 25, 2532; Gonzalgo, M.L. and Jones, P.A. (1997), Nucl. Acids Res. 25, 2529; Grigg, S. and Clark, S. (1994), Bioessays 16, 431; Zeschnik, M. et al. (1997), Human Molecular Genetics 6, 387; Teil, R. et al. (1994), Nucl. Acids Res. 22, 695; Martin, V. et al. (1995), Gene 157, 261; WO 97/46705, WO 95/15373 und WO 97/45560.

Gemeinsamkeiten zwischen Promotoren bestehen nicht nur im Vorkommen von TATA- oder GC-Boxen sondern auch darin, für welche Transkriptionsfaktoren sie Bindestellen besitzen und in welchem Abstand diese sich zueinander befinden. Die existierenden Bindestellen für ein bestimmtes Protein stimmen in ihrer Sequenz nicht vollständig überein, es finden sich aber konservierte Folgen von mindestens 4 Basen, die durch das Einfügen von "Wobbles", d. h. Positionen, an denen sich jeweils unterschiedliche Basen befinden, noch verlängert werden können. Des weiteren liegen diese Bindestellen in bestimmten Abständen zueinander vor.

Eine Übersicht über den Stand der Technik in der Oligomer Array Herstellung läßt sich auch einer im Januar 1999 erschienen Sonderausgabe von Nature Genetics (Nature Genetics Supplement, Volume 21, January 1999) und der dort zitierten Literatur entnehmen.

Matrix-assistierte Laser Desorptions/Ionisations Massenspektrometrie (MALDI) ist eine neue, sehr leistungsfähige Entwicklung für die Analyse von Biomolekülen (Karas, M. and Hillenkamp, F. 1988. Laser desorption ionization of proteins with molecular masses exceeding 10.000 daltons. Anal. Chem. 60: 2299-2301). Ein Analytmolekül wird in eine im UV absorbierende Matrix eingebettet. Durch einen kurzen Laserpuls wird die Matrix ins Vakuum verdampft und das Analyt so unfragmentiert in die Gasphase befördert. Eine angelegte Spannung beschleunigt die Ionen in ein feldfreies Flugrohr. Auf Grund ihrer verschiedenen Massen werden Ionen unterschiedlich stark beschleunigt. Kleinere Ionen erreichen den Detektor früher als größere und die Flugzeit wird in die Masse der Ionen umgerechnet.

Für die Abtastung eines immobilisierten DNA-Arrays sind vielfach fluoreszent markierte Sonden verwendet worden. Besonders geeignet sind für die Fluoreszenzmarkierung ist das einfache Anbringen von Cy3 und Cy5 Farbstoffen am 5'OH der jeweiligen Sonde. Die Detektion der Fluoreszenz der hybridisierten Sonden erfolgt beispielsweise über ein Konfokalmikroskop. Die Farbstoffe Cy3 und Cy5 sind, neben vielen anderen, kommerziell erhältlich.

Methylasen, die Cytosinbasen in bestimmenten Sequenzkontexten methylieren, sind bekannt und zum Teil auch kommerziell erhältlich. Sss1 Methylase beispielsweise methyliert Cytosin im Sequenzkontext CpG (siehe z.B. Renbaum, P. et al. (1990), Nucleic Acids Res. 18, 1145) und ist z. B. Bei New England Biolabs erhältlich, ebenso wie andere Methylasen wie Alul, BamH1 oder HaeIII.

Ausschließlich durch Bisulfit-Behandlung, Amplifikation und nachfolgendes Sequenzieren oder Hybridisieren, wie es Stand der Technik ist, lassen sich methylierte Cytosinpositionen nicht mit Sicherheit nachweisen. Wird an der betreffenden Position ein Thymin statt eines Cytosins nachgewiesen, so kann es sich um eine Mutation gegenüber der herangezogenen Vergleichssequenz, aber auch um ein in der genomischen Proben-DNA nicht methyliertes Cytosin handeln, das durch die Bisulfit behandlung zuerst in Uracil und bei der Amplifikation schließlich in Thymin im Sequenzkontext umgewandelt wurde, handeln. Aufgabe der vorliegenden Erfindung ist es daher dieses Problem zu lösen.

Die Aufgabe wird dadurch gelöst, daß ein Verfahren zur Unterscheidung von 5-Position Methylierungsänderungen von Cytosin-Basen und Cytosin-zu-Thymin Mutationen und zum Nachweis von single nucleotide polymorphisms (SNPs) oder Punktmutationen in genomischer DNA geschaffen wird, bei dem man
a) eine genomische DNA-Probe mit Sulfit oder Disulfit oder einer anderen Chemikalie derart behandelt, daß alle nicht an der 5-Position der Base methylierten Cytosinbasen derart verändert werden, so daß eine dem Basenpaarungsverhalten nach unterschiedliche Base entsteht, während die an der 5-Position methylierten Cytosine unverändert bleiben und
b) ein Aliquot der selben genomischen DNA-Probe vor der chemischen Behandlung nach a) mit Sss1 oder einer anderen Methyltransferase quantitativ aufmethyliert und
c) die beiden so behandelten DNA-Proben mittels der gleichen analytischen Methode auf die Präsenz von Cytosin untersucht und
d) die ermittelten Cytosin Positionen mit einer Referenz DNA-Sequenz abgeglichen werden.

Die vorliegende Erfindung löst die Aufgabe also dadurch, daß ein aufmethyliertes Aliquot derselben Probe gleichermaßen behandelt wird und die erhaltenen Sequenzinformationen mit der Sequenz einer Referenz-DNA abgeglichen werden, wobei eine Unterscheidung zwischen Mutationen und Cytosin Methylierung möglich wird. Zudem ist es auch möglich, im Rahmen dieses Verfahrens neue Mutationen und Polymorphismen, insbesondere C-T Mutationen, aufzufinden.

Erfindungsgemäß bevorzugt ist ein Verfahren, wobei man über den Vergleich der einzelnen Cytosin-Positionen aus den beiden unterschiedlich behandelten Proben mit der Referenz-Sequenz ermittelt, ob an einer bestimmten Position Cytosin nicht nachweisbar ist und ob dies darauf beruht, daß das Cytosin in der genomischen DNA unmethyliert vorliegt oder durch eine Mutation oder einen Polymorphismus verändert vorliegt und somit in der genomischen DNA nicht vorhanden ist.

Erfindungsgemäß ist ferner ein Verfahren, welches dadurch gekennzeichnet ist, daß man die beiden DNA-Proben oder Teile dieser DNA-Proben vor dem Nachweis der Base Cytosin mit einem cyclischen Prozess, nämlich der Polymerase Kettenreaktion oder einem vergleichbaren Prozess amplifiziert.

Bevorzugt ist dabei, daß man in einem Amplifikations-Ansatz mehr als 10 verschiedene Fragmente der behandelten genomischen DNAs erzeugt.

Bevorzugt ist es ferner, daß man zur Amplifikation der genomischen DNA-Proben solche Primer verwendet, welche für die Genregulation wichtige sogenannte Konsensus-Sequenzen oder solche Sequenzen enthalten und die damit überwiegend an regulative oder kodierende Sequenzen binden.

Besonders ist es erfindungsgemäß bevorzugt, daß man Cytosin im spezifischen Kontext 5'-CpG-3' detektiert.

Weiterhin ist es bevorzugt, daß man die DNA bei der Amplifikation durch Einbau von mit einer detektierbaren Markierung versehenen Nukleotidbausteinen oder Oligonukleotiden insgesamt mit einer oder mehreren detektierbaren Markierung(en) versieht.

Insbesondere bevorzugt ist es, daß die Detektion der Markierung durch Fluoreszenz oder Chemoluminiszenz erfolgt.

Beim erfindungsgemäßen Verfahren ist ferner bevorzugt, daß man Cytosin über Hybridisierung mit für "Sequenzkontext-Cytosin-Sequenzkontext" spezifischen Oligomeren nachweist, welche in einer definierten Anordnung auf einer oder mehreren Oberflächen fixiert sind.

Hierbei ist es bevorzugt, daß man für jedes in seinem sequenzspezifischen Kontext nachzuweisende Cytosin mindestens ein zu dem Sequenzkontext komplementäres Oligomer auf der Oberfläche fixiert, welches das zum nachzuweisenden Cytosin komplementäre Guanin enthält und ein weiteres Oligomer, welches an der Stelle des nachzuweisenden Cytosins die Base enthält, welche zu der Base komplementär ist, in welche nicht methylierte Cytosine durch die chemische Behandlung umgewandelt werden.

Weiterhin ist hierbei bevorzugt, daß man für nachzuweisende Cytosin-Positionen solche Oligomere fixiert, die jeweils an die methylierte und unmethylierte Position sowohl auf Plus- als auch auf Minus-Strang spezifisch binden oder/und an die je durch Amplifikation entstehenden komplementären Stränge spezifisch hybridisieren.

Hierbei ist weiterhin bevorzugt, daß man auf der Oberfläche weitere Oligomere fixiert, welche jeweils an die Sequenz "Sequenzkontext-Thymin-Sequenzkontext" spezifisch binden und/oder welche Cytosin und die durch chemische Behandlung entstandene Base im Plus-Strang, Minus-Strang und den je durch Amplifikation entstehenden komplementären Strängen entstehenden Strängen nachweisen.

Es ist erfindungsgemäß weiterhin bevorzugt, daß man von den Oligomeren an Punkten der Oberfläche(n) Signale detektiert, die für die in der originalen genomischen Probe methylierten, oder unmethylierten oder mutierten spezifisch sind.

Dabei ist insbeondere bevorzugt, daß man durch den Vergleich der detektierten Signale der absolute Grad der Methylierung und/oder die Homo- bzw. Heterozygotie ermittelt.

Erfindungsgemäß ist ferner das Verfahren, wobei man die amplifizierten Fragmente der beiden Proben auf je einer Oberfläche fixiert und mit einer nachweisbaren Markierung versehene, sequenzspezifische Oligomere auf diese Oberflächen hybridisiert.

Bevorzugt ist es, daß man die Analyse der hybridisierten sequenzspezifischen Oligomere mittels Massenspektrometrie und bevorzugt einem MALDI Massenspektrometer durchführt.

Es ist weiterhin bevorzugt, daß man die Analyse der hybridisierten sequenzspezifischen Oligomere mittels Fluoreszenz oder Chemoluminiszenz durchführt.

Eine besonders bevorzugte Variante des Erfindungsgemäßen Verfahrens ist es, daß der Nachweis von Cytosin im Sequenzkontext durch eine Polymerase-Reaktion, welche spezifisch bei Erreichen einer Cytosin-Base im Templat gestoppt wird, und Längenmessung der entstehenden Fragmente erfolgt.

Hierbei ist insbesondere bevorzugt, daß man die Oligomere, die zum Starten der primer-abhängigen Polymerase-Reaktion benutzt werden, je unterschiedliche Sequenz an unterschiedlichen Orten auf einer Oberfläche fixiert und die Polymerase-Reaktion auf dieser Oberfläche erfolgt.

Dabei ist weiterhin bevorzugt, daß man die Oligomere, die zum Starten der primer-abhängigen Polymerase-Reaktion benutzt werden, durch eine chemische Reaktion oder durch Licht von der Oberfläche ablöst.

Erfindungsgemäße ist weiterhin bevorzugt, daß man zur Termination der Polymerasereaktion an der Position eines Cytosins oder - im Gegenstrang - eines Guanins einen Nukleotidbaustein verwendet, welcher über eine chemische Modifikation auch eine Detektion zum Beispiel durch Fluoreszenz, Chemoluminiszenz oder das Binden eines Antikörpers erlaubt.

Außerdem ist bevorzugt, daß man die Detektion der Termination an der Position eines Cytosins oder - im Gegenstrang - eines Guanins über eine Längenmessung der entstehenden Fragmente durch Gelelektorphorese, insbesondere Kapillarelektrophorese durchführt.

Weiterhin ist dabei bevorzugt, daß man die Längenmessung der entstehenden Fragmente durch massenspektrometrische Analyse und bevorzugt in einem MALDI Massenspektrometer durchführt.

Das erfindungsgemäße Verfahren ist ferner, dadurch gekennzeichnet, daß die Referenz DNA-Sequenz aus einer Datenbank, nämlich aus dem Humangenom-Projekt, stammt.

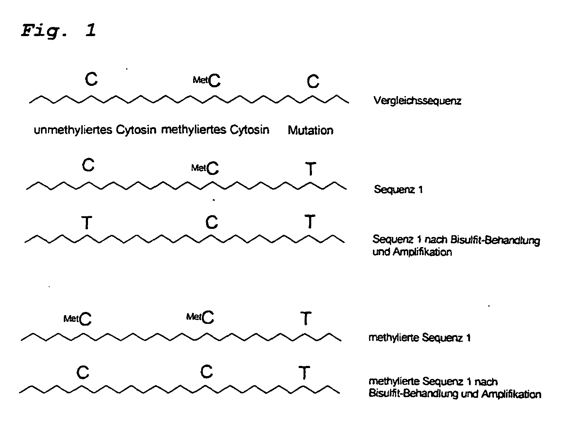

Das beschriebene Verfahren dient zur Unterscheidung von 5-Methylcytosin-Positionen und Punktmutationen/Polymorphismen in genomischer DNA, kann jedoch auch zum Auffinden und zum Nachweis von Punktmutationen/Polymorphismen genutzt werden. Abb. 1 faßt das Verfahren am Beispiel einer beliebigen Sequenz 1 zusammmen.

Zur Durchführung des erfindungsgemäßen Verfahrens werden die folgenden Schritte ausgeführt:

Eine genomische DNA-Probe wird derart chemisch behandelt, daß alle nicht an der 5-Position methylierten Cytosinbasen derart verändert werden, daß eine dem Basenpaarungsverhalten nach unterschiedliche Base entsteht, während die an der 5-Position methylierten Cytosine unverändert bleiben. Bevorzugt wird in diesem Schritt eine Behandlung mit einer Bisulfitlösung (=Hydrogensulfit, Disulfit) und anschließende alkalische Hydrolyse durchgeführt. Diese Behandlung führt zu einer Umwandlung der nicht methylierten Cytosinbasen in Uracil, dessen Basenpaarungsverhalten dem Thymin entspricht, als das die betreffenden Positionen nach der Amplifikation auch vorliegen.

Im zweiten Schritt wird ein Aliquot der selben genomischen DNA-Probe vor der oben beschriebenen chemischen Behandlung mit Sss1 oder einer anderen Methyltransferase aufmethyliert. Diese Methylierung führt dazu, daß alle Cytosinbasen im Sequenzkontext CG der DNA-Probe oder mit anderen Methyltransferasen sämtliche Cytosinbasen der DNA-Probe in 5-Methylcytosin umgewandelt werden und damit bei der chemischen Behandlung keine Umwandlung in Thymin mehr erfolgen kann. In einer besonders bevorzugten Variante des Verfahrens werden beide DNA-Proben nach den beschriebenen Vorbehandlungsschritten amplifiziert, wiederum bevorzugt wird dies mittels PCR durchgeführt.

In einem dritten Schritt des Verfahrens werden die beiden behandelten DNA-Proben mittels der gleichen analytischen Methode auf die Präsenz von Cytosin untersucht. Die ermittelten Cytosin Positionen werden mit denen einer Referenz DNA-Sequenz abgeglichen. Diese Referenz DNA Sequenz kann prinzipiell beliebig gewählt werden, kann jedoch mit der untersuchten Probe nicht identisch sein. Bevorzugt stammt die Referenzsequenz aus einer Datenbank, wie sie derzeit beispielsweise im Rahmen des Humangenomprojektes entstehen oder bereits vorhanden sind. Der Abgleich mit der Referenz-DNA kann nun je nach detektierten Cytosinbasen für eine gegebene Position in der Probe zu den folgenden Ergebnissen führen: Wird sowohl in der aufmethylierten Probe als auch in der nicht aufmethylierten Probe ein Cytosin nachgewiesen, so liegt dieses Cytosin in der genomischen DNA methyliert vor. Wird nur in der aufmethylierten Probe ein Cytosin nachgewiesen ind in der nicht aufmethylierten Probe ein Thymin, so liegt in der genomischen DNA ein nicht methyliertes Cytosin vor. Wird dagegen in beiden Proben ein Thymin nachgewiesen und ist aber in der Referenz-Sequenz an gleicher Position ein Cytosin vorhanden, so liegt eine C-T Punktmutation oder ein "Single Nucleotide Polymorphism" (SNP) vor.

In einer bevorzugten Variante des Verfahrens wird die Amplifikation im zweiten Schritt so durchgeführt, daß in einem Amplifikationsansatz mehr als 10 unterschiedliche Fragmente erzeugt werden. Dies kann bevorzugt durch Verwendung von Primern erfolgen, welche für die Genregulation wichtige sogenannte Konsensus-Sequenzen oder solche Sequenzen enthalten und die damit überwiegend an regulative oder kodierende Sequenzen binden. Bei dieser Amplifikation werden die Produkte bevorzugt durch Einbau von mit einer detektierbaren Markierung versehenen Nukleotidbausteinen oder Oligonukleotiden (z. B. Primer) insgesamt mit einer oder mehreren detektierbaren Markierung(en) versehen, wobei eine Detektion der Markierung besonders bevorzugt durch Fluoreszenz oder Chemoluminiszenz erfolgt.

Die Untersuchung der Cytosin Positionen erfolgt in einer besonders bevorzugten Variante des Verfahrens derart, daß Cytosin ausschließlich im spezifischen Kontext 5'-CpG-3' detektiert wird. Dies kann bevorzugt durch einen Nachweis des Cytosins über Hybridisierung mit für "Sequenzkontext-Cytosin-Sequenzkontext" spezifischen Oligomeren durchgeführt werden, welche in einer definierten Anordnung auf einer oder mehreren Oberflächen fixiert sind. In einer bsonders bevorzugten Variante des Verfahrens ist für jedes in seinem sequenzspezifischen Kontext nachzuweisende Cytosin mindestens ein zu dem Sequenzkontext komplementäres Oligomer auf der Oberfläche fixiert, welches das zum nachzuweisenden Cytosin komplementäre Guanin enthält und ein weiteres Oligomer, welches an der Stelle des nachzuweisenden Cytosins die Base enthält, welche zu der Base komplementär ist, in welche nicht methylierte Cytosine durch die chemische Behandlung umgewandelt werden. In einer besonders bevorzugten Variante des Verfahrens handelt es sich hier, falls eine Bisulfit-Behandlung durchgeführt wird, um die dem Thymin komplementäre Base Adenin.

In einer weiteren besonders bevorzugten Variante des Verfahrens werden für nachzuweisende Cytosin-Positionen solche Oligomere an der oder den Oberfläche(n) fixiert, die jeweils an die methylierte und unmethylierte Cytosin-Position sowohl auf den Plus- als auch auf dem Minus-Strang spezifisch binden oder/und an die jeweils durch Amplifikation entstehenden komplementären Stränge spezifisch hybridisieren.

In einer weiteren besonders bevorzugten Variante des Verfahrens werden auf der oder den Oberfläche(n) weitere Oligomere fixiert, welche jeweils an die Sequenz "Sequenzkontext-Thymin-Sequenzkontext" spezifisch binden und/oder welche Cytosin und die durch chemische Behandlung entstandene Base im Plus-Strang, Minus-Strang und den je durch Amplifikation entstehenden komplementären Strängen entstehenden Strängen nachweisen.

In einer besonders bevorzugten Variante des Verfahrens werden an den Punkten der Oberfläche(n), an denen die Oligomere fixiert sind, Signale detektiert, die für die in der originalen genomischen Probe methylierten, oder unmethylierten oder mutierten spezifisch sind. In einer bevorzugten Variante.des Verfahrens sind die detektierten Signale quantifizierbar, so daß der absolute Grad der Methylierung und/oder die Homo- bzw. Heterozygotie ermittelbar sind.

In einer weiteren bevorzugten Variante des Verfahrens werden die amplifizierten Fragmente der beiden Proben, aufmethyliert und nicht aufmethyliert, auf je einer Oberfläche fixiert und mit einer nachweisbaren Markierung versehene, sequenzspezifische Oligomere auf diesen Oberflächen an die beiden Proben hybridisiert. In einer besonders bevorzugten Variante des Verfahrens werden die hybridisierten sequenzspezifischen Oligomere mittels Massenspektrometrie und bevorzugt einem MALDI Massenspektrometer nachgewiesen. In einer weiteren, besonders bevorzugten Variante des Verfahrens erfolgt die Analyse der hybridisierten sequenzspezifischen Oligomere mittels deren Fluoreszenz oder Chemoluminiszenz.

In einer weiteren bevorzugten Variante des Verfahrens wird der Nachweis von Cytosin im Sequenzkontext durch eine Polymerase-Reaktion, welche spezifisch bei Erreichen einer Cytosin-Base im Templat gestoppt wird, und Längenmessung der entstehenden Fragmente durchgeführt. Dabei können die Oligomere, die zum Starten der primer-abhängigen Polymerase-Reaktion benutzt werden, bevorzugt je unterschiedliche Sequenz an unterschiedlichen Orten auf einer Oberfläche fixiert sein und die Polymerase-Reaktion kann bevorzugt auf dieser Oberfläche erfolgen. In einer weiteren bsonders bevorzugten Variante des Verfahrens können die Oligomere, die zum Starten der primer-abhängigen Polymerase-Reaktion benutzt werden, durch eine chemische Reaktion oder durch Licht von der Oberfläche abgelöst werden. Zur Termination der Polymerasereaktion an der Position eines Cytosins oder - im Gegenstrang - eines Guanins wird bevorzugt ein Nukleotidbaustein verwendet, welcher über eine chemische Modifikation auch eine Detektion zum Beispiel durch Fluoreszenz, Chemoluminiszenz oder das Binden eines Antikörpers erlaubt. Auch kann die Detektion der Termination an der Position eines Cytosins oder - im Gegenstrang - eines Guanins über eine Längenmessung der entstehenden Fragmente durch Gelelektorphorese, insbesondere Kapillarelektrophorese erfolgen. In einer weitern bevorzugten Varainte des Verfahrens erfolgt die Längenmessung der enstehenden Fragmente durch massenspektrometrische Analyse und bevorzugt in einem MALDI Massenspektrometer.

Zur Durchführung des Verfahrens kann ein Kit verwendet werden, der Referenz-DNA und/oder Chemikalien und Hilfsmittel zur Durchführung der Bisulfit-Reaktion und/oder der Amplifikation und/oder eine Methyltransferase und/oder Dokumentation zur Durchführung des Verfahrens enthält.

Das erfindungsgemäße Verfahren wird an Hand der beigefügten Abbildung erläutert.

In Figur 1 sind Punktmutationen von Cytosin zu Thymin als Thymin sowohl in der Bisulfit-behandelten und amplifizierten als auch in der zuvor methylierten, Bisulfit-behandelten und amplifizierten DNA nachweisbar. Methylierte Cytosine werden in beiden Fällen als Cytosin nachgewiesen, dagegen werden unmethylierte Cytosine nur in der nicht methylierten Probe als T und in der methylierten Probe als C nachgewiesen.

Das folgende Beispiel erläutert die Erfindung:

### Beispiel:

Als Beispiel soll die folgende DNA Sequenz dienen, die ein potentiell methyliertes CG Dinukleotid oder eine Punktmutation CG nach TG (C-SNP) enthält:
Auschnitt I) aus der in der Datenbank Genbank eingetragenen genomischen Sequenz mit der Accession Nummer AL031228 von Position 117606 bis Position 118388.

I)
AAAAGGGTGGGGCTTCTATGGGGGGTCCTCTGTGTGGCCGCTGGCTTGGGTATTG
GGAAGCCGGGGGTATGGCAGGGTGGGCAAGGGGATGGGGTATTGACAGTTTTGGAGG
TGATGCCAGCCAGGTTGGGGGCCCACCTCTGACCTTGCTTCACTTCTGCAGGGCCAG
GATGGTGCTAAGGGTGACCGAGGCGAGGATGGTGAGCCAGGACAGCCTGTGAGTGCC

TGGTGACCCCACCACCCCCCTGAGCCCAAGCCTCATCCTCTTTACCCCTCTTCTGTG
CCCCACTCCTGAGGGGTCCCTTGGCTGGAGGATAAACACTCAGCCACCCCAATTCCT
CTCTCCCTAGGGATCCCCTGGTCCCACCGGGGAGAATGGACCCCCAGGGCCACTTGG
AAAGCGAGTAAGTGAGGTGGACCCCTGAGACCTTGGGAGGCAGTCCCTGGCTGTGT
GGGTGGAGGCTGGGCAATGGCAGGTGGGATGGGTGGGGAGGTGCCTGGTGTCTGCAT
TGCCCTGGGTGTGTGTGTGTGCAGGAGCTGGTGGGTTTAAGGGC/TGTGTGGTATCT
CATTGCCCGGGGCAGGGTGTGTGTGCAGGAGCTGGTGGGTTTAAGGGTATGCGGTAT
CTCATTGCACTGGGCGAGTGTGTGTGCAGGAGCTGGTGGGTTGATGGGTGTGCGGTA
TCTCGGGCATGTTTGTTCCTGGGTTCTGGTGTGTATGTTTTCACCAGGGGTAGTGGT
GGTTACTGACAAAGCAGAATGGAAACTGGAGGAGGGGCTGGCCAGCTTTTCTGTGGG
CCAGGGGTGAACCTTTTTAGTTTCTGGGGCAGGAGACGGCCACCAGGTAGGGTGTG
GGCAAGTGGCCCTTCACCAAATGTACAGACTACCCAGTATTTTCACAACTGTCACAG
CTGTATCTGTTCTGCACATCTGTGAATCGGCCCTCGGCGCGTGTCCCTGTGTATGCA
CGTGTGTGTGTGCATGTGTATGTGTGTGTCTAGGACAGGAAGGGGGAAGAGTTGAGC
CTGGCTGCCCACGGCCTCATGTGCTCTTCCTTCCCACTCCACCTGCAGGGTCCTGCT
GGCT

Das unterlegte C/T kennzeichnet den C-SNP, der sich in der Sequenz mit der Accession Nummer AL031228 an Position 117606 befindet.

Nachfolgend ist die Sequenz A) der methylierten und Bisulfit behandelten DNA dargestellt.

A)

AAAAGGGTGGGTTTTTATGGGGGGTTTTTGTGTGGTCGTTGGGTTTGGGTATTG
GGAAGTCGGGGGTATGGTAGGGTGGGTAAGGGGATGGGTATTGATAGTTTTGGAGG
TGATGTTAGTTAGGTTGGGGGTTTATTTTTGATTTTGTTTTATTTTTGTAGGGTTAG
GATGGTGTTAAGGGTGATCGAGGCGAGGATGGTGAGTTAGGATAGTTTGTGAGTGTT
TGGTGATTTTATTATTTTTTTGAGTTTAAGTTTTATTTTTTTATTTTTTTTTTGTG
TTTTATTTTTGAGGGGTTTTTTGGTTGGAGGATAAATATTTAGTTATTTTAATTTTT
TTTTTTTTAGGGATTTTTTGGTTTATCGGGAGAATGGATTTTTAGGGTTATTTGG
AAAGCGAGTAAGTGAGGTGGATTTTTGAGATTTTGGGAGGTAGTTTTTGGGTTGTGT
GGGTGGAGGTTGGGTAATGGTAGGTGGGATGGGTGGGGAGGTGTTTGGTGTTTGTAT
TGTTTTGGGTGTGTGTGTGTGTAGGAGTTGGTGGGTTTAAGGGC/TGTGTGGTATTT
TATTGTTCGGGGTAGGGTGTGTGTGTAGGAGTTGGTGGGTTTAAGGGTATGCGGTAT

TTTATTGTATTGGGCGAGTGTGTGTAGGAGTTGGTGGGTTGATGGGTGTGCGGTA
TTTCGGGTATGTTTGTTTTTGGGTTTTGGTGTGTATGTTTTTATTAGGGGTAGTGGT
GGTTATTGATAAAGTAGAATGGAAATTGGAGGAGGGGTTGGTTAGTTTTTTTGTGGG
TTAGGGGTGAATTTTTTTAGTTTTTGGGGTAGGAGACGGGTTATTAGGTAGGGTGTG
GGTAAGTGGTTTTTTATTAAATGTATAGATTATTTAGTATTTTTATAATTGTTATAG
TTGTATTTGTTTTGTATATTTGTGAATCGGTTTTCGGCGCGTGTTTTTGTGTATGTA
CGTGTGTGTGTGTATGTGTATGTGTGTGTTTAGGATAGGAAGGGGGAAGAGTTGAGT
TTGGTTGTTTACGGTTTTATGTGTTTTTTTTTTTTATTTTATTTGTAGGGTTTTGTT
GGTT

Nachfolgend ist die Sequenz B) der nichtmethylierten und Bisulfit behandelten DNA dargestellt.

B)
AAAAGGGTGGGTTTTTATGGGGGGTTTTTGTGGTTGTTGGGTTTGGGTATTG
GGAAGTTGGGGGTATGGTAGGGTGGGTAAGGGGATGGGGTATTGATAGTTTTGGAGG
TGATGTTAGTTAGGTTGGGGGTTTATTTTTGATTTTGTTTTATTTTTGTAGGGTTAG
GATGGTGTTAAGGGTGATTGAGGTGAGGATGGTGAGTTAGGATAGTTTGTGAGTGTT
TGGTGATTTTATTATTTTTTTGAGTTTAAGTTTTATTTTTTTATTTTTTTTTTGTG
TTTTATTTTTGAGGGGTTTTTTGGTTGGAGGATAAATATTTAGTTATTTTAATTTTT
TTTTTTTTAGGGATTTTTTGGTTTTATTGGGGAGAATGGATTTTTAGGGTTATTTGG
AAAGTGAGTAAGTGAGGTGGATTTTTGAGATTTTGGGAGGTAGTTTTTGGGTTGTGT
GGGTGGAGGTTGGGTAATGGTAGGTGGGATGGGTGGGGAGGTGTTTGGTGTTTGTAT
TGTTTTGGGTGTGTGTGTGTGTAGGAGTTGGTGGGTTTAAGGGC/TGTGTGGTATTT
TATTGTTTGGGGTAGGGTGTGTGTGTAGGAGTTGGTGGGTTTAAGGGTATGTGGTAT
TTTATTGTATTGGGTGAGTGTGTGTGTAGGAGTTGGTGGGTTGATGGGTGTGTGGTA
TTTTGGGTATGTTTGTTTTTGGGTTTTGGTGTGTATGTTTTTATTAGGGGTAGTGGT
GGTTATTGATAAAGTAGAATGGAAATTGGAGGAGGGGTTGGTTAGTTTTTTTGTGGG
TTAGGGGTGAATTTTTTTAGTTTTTGGGGTAGGAGATGGGTTATTAGGTAGGGTGTG
GGTAAGTGGTTTTTTATTAAATGTATAGATTATTTAGTATTTTTATAATTGTTATAG
TTGTATTTGTTTTGTATATTTGTGAATTGGTTTTTGGTGTGTGTTTTTGTGTATGTA
TGTGTGTGTGTGTATGTGTATGTGTGTGTTTAGGATAGGAAGGGGGAAGAGTTGAGT
TTGGTTGTTTATGGTTTTATGTGTTTTTTTTTTTTTATTTTATTTGTAGGGTTTTGTT
GGTT

Im amplifizierten Fragment 1 mit der Länge 832 bp (Positionen 1 bis 832) befinden sich der C-SNP an Position 557, im amplifizierten Fragment 2 mit der Länge 783 (Positionen 303 bis 1085) befindet sich der C-SNP an Position 255. Für Fragment 1 sind die Primer in Fettdruck, für das Fragment 2 sind die Primer in Fettdruck und unterstrichen dargestellt .

Genomische DNA wird mit dem Restriktionsenzym Mss1 (Fermentas) geschnitten und anschließend mit dem Enzym Sss1 (CpG Methylase, BioLabs) aufmethyliert. Die Bisulfitreaktion wird in an sich bekannter Weise durchgeführt. In der anschließenden Polymerasereaktion wird das Gen COL11A2 auf Chromosom 6p21 amplifiziert. Die Amplifikation wird nach dem allgemeinen PCR-Protokoll mit dem Primerpaar **AAAAGGGTGGGGTTTTTAT, TCTCCTACCCCAAAAACTAA** oder mit dem Primerpaar **TTTTTGGTTGGAGGATAAATA****,** **AACCAACAAAACCCTACAAA** durchgeführt. Hierbei wurden beide oder nur ein Primer der jeweiligen Primerpaare mit Cy5 markiert.

### PCR Ansatz (20µL):

1µL DNA (10 ng), je 2µL (4x25mM) dNTPs, 0,2 µL (1 unit) Taq(Hot Star Taq®, Qiagen), 2µL PCR-Puffer (10x, Qiagen), je 1 µL Cy5 markierte Primer (6,25 pmoL/µL)
Dadurch werden zwei DNA Fragmente amplifiziert, die in A) dargestellt sind.

Genomische DNA wird mit dem Restriktionsenzym Mss1 (Fermentas) geschnitten. Die Bisulfitreaktion wird gemäß dem aufgeführten Stand der Technik durchgeführt. In der anschließenden Polymerasereaktion wird das Gen COL11A2 auf Chromosom 6p21 amplifiziert. Die Amplifikation wird nach dem allgemeinen PCR-Protokoll mit dem Primerpaar **AAAAGGGTGGGGTTTTTAT, TCTCCTACCCCAAAAACTAA oder** mit dem Primerpaar **TTTTTGGTTGGAGGATAAATA****,** **AACCAACAAAACCCTACAAA** durchgeführt. Hierbei wurden beide oder nur ein Primer der jeweiligen Primerpaare mit Cy5 markiert.

### PCR Ansatz (20µL):

1µL DNA (10 ng), je 2µL (4x25mM) dNTPs, 0,2 µL (1 unit) Taq(Hot Star Taq®, Qiagen), 2µL PCR-Puffer (10x, Qiagen), je 1 µL Cy5 markierte Primer (6,25 pmoL/µL) Dadurch werden zwei DNA Fragmente amplifiziert, die in B dargestellt sind.

Für die Analyse des in I)dargestellten CG oder TG Dinukleotides wurden folgende Oligonukleotide, die die Sequenzen TTTAAGGGCGTGTGGTAT und TTTAAGGGTGTGTGGTAT beinhalten, an einer Glasoberfläche fixiert. In getrennten Experimenten werden Glasträger mit dem DNA-Fragment 1 amplifiziert aus nichtmethylierter Bisulfit-behandelter DNA und mit dem DNA-Fragment 1 amplifiziert aus methylierter Bisulfit behandelter DNA und/oder mit dem DNA-Fragment 2 amplifiziert aus nichtmethylierter Bisulfit-behandelter DNA und mit dem DNA-Fragment 2 amplifiziert aus methylierter Bisulfit behandelter DNA und in an sich bekannter Weise hybridisiert. Dieses Verfahren ist vollständig automatisiert.

Ausgehend von der genomischen Sequenz CTGGTGGGTTTAAGGGC/TGTGTGGTATCTC sind die möglichen Hybridisierungergebnisse denkbar, die es ermöglichen den Methylierungszustand oder eine Punktmutation im CG Dinukleotid nachzuweisen:
nicht-methylierte DNA (bisulfit behandelt)
Fall 1) CTGGTGGGTTTAAGGGCGTGTGGTATCTC methyliertes C
Fall 2) TTGGTGGGTTTAAGGGTGTGTGGTATTTT nicht methyliertes C
Fall 3) TTGGTGGGTTTAAGGGTGTGTGGTATTTT Punktmutation/SNP methylierte DNA (bisulfit behandelt)

Fall 1) CTGGTGGGTTTAAGGGCGTGTGGTATCTC methyliertes C
Fall 2) TTGGTGGGTTTAAGGGCGTGTGGTATTTT nicht methyliertes C
Fall 3) TTGGTGGGTTTAAGGGTGTGTGGTATTTT Punktmutation/SNP

Fall 1: Liegt in der genomischen Sequenz ein methyliertes Cytosin vor, wird in der nicht-methylierten Bisulfit-behandelten DNA und in der methylierten Bisulfit-behandelten DNA ein Cytosin nachgewiesen.

Fall 2: Liegt in der genomischen Sequenz ein nichtmethyliertes Cytosin vor, wird in der nicht-methylierten Bisulfit-behandelten DNA ein Thymin nachgewiesen und in der methylierten Bisulfit-behandelten DNA ein Cytosin nachgewiesen.

Fall 3: Liegt in der genomischen Sequenz eine Punktmutation (C-SNP) vor, wird in der nicht-methylierten Bisulfit-behandelten DNA und in der methylierten Bisulfitbehandelt DNA ein Thymin nachgewiesen.

## Patentansprüche

1. Verfahren zur Unterscheidung von 5-Position Methylierungsänderungen von Cytosin-Basen und Cytosin-zu-Thymin Mutationen und zum Nachweis von single nucleotide polymorphisms (SNPs) oder Punktmutationen in genomischer DNA, **dadurch gekennzeichnet, daß** man
a) eine genomische DNA-Probe mit Sulfit oder Disulfit derart behandelt, daß alle nicht an der 5-Position der Base methylierten Cytosinbasen derart verändert werden, so daß eine dem Basenpaarungsverhalten nach unterschiedliche Base entsteht, während die an der 5-Position methylierten Cytosine unverändert bleiben und
b) ein Aliquot der selben genomischen DNA-Probe vor der chemischen Behandlung nach a) mit SSS1 oder einer anderen Methyltransferase quantitativ aufmethyliert und
c) die beiden so behandelten DNA-Proben mittels der gleichen analytischen Methode auf die Präsenz von Cytosin untersucht und
d) die ermittelten Cytosin Positionen mit einer Referenz DNA-Sequenz abgeglichen werden und
e) über den Vergleich der einzelnen Cytosin-Positionen aus den beiden unterschiedlich behandelten Proben mit der Referenz-Sequenz wird ermittelt, ob an einer bestimmten Position Cytosin nicht nachweisbar ist und ob dies darauf beruht, daß das Cytosin in der genomischen DNA unmethyliert vorliegt oder durch eine Mutation oder einen Polymorphismus verändert vorliegt und somit in der genomischen DNA nicht vorhanden ist.

2. Verfahren nach einem der vorangehenden Ansprüche, **dadurch gekennzeichnet, daß** man die beiden DNA-Proben oder Teile dieser DNA-Proben vor dem Nachweis der Base Cytosin mit einem cyclischen Prozess, nämlich der polymerase Kettenreaktion oder einem vergleichbaren Prozess amplifiziert.

3. Verfahren nach Anspruch 2, **dadurch gekennzeichnet, daß** man in einem Amplifikations-Ansatz mehr als 10 verschiedene Fragmente der behandelten genomischen DNAs erzeugt.

4. Verfahren nach einem der vorangehenden Ansprüche, **dadurch gekennzeichnet, daß** man zur Amplifikation der genomischen DNA-Proben solche Primer verwendet, welche für die Genregulation wichtige sogenannte Konsensus-Sequenzen oder solche Sequenzen enthalten und die damit überwiegend an regulative oder kodierende Sequenzen binden.

5. Verfahren nach einem der vorangehenden Ansprüche, **dadurch gekennzeichnet, daß** man Cytosin im spezifischen Kontext 5'-CpG-3' detektiert.

6. Verfahren nach einem der vorangehenden Ansprüche, **dadurch gekennzeichnet, daß** man die DNA bei der Amplifikation durch Einbau von mit einer detektierbaren Markierung versehenen Nukleotidbausteinen oder Oligonukleotiden insgesamt mit einer oder mehreren detektierbaren Markierung(en) versieht.

7. Verfahren nach Anspruch 6 **dadurch gekennzeichnet, daß** die Detektion der Markierung durch Fluoreszenz oder Chemoluminiszenz erfolgt.

8. Verfahren nach einem der vorangehenden Ansprüche, **dadurch gekennzeichnet, daß** man Cytosin über Hybridisierung mit für "Sequenzkontext-Cytosin-Sequenzkontext" spezifischen Oligomeren nachweist, welche in einer definierten Anordnung auf einer oder mehreren Oberflächen fixiert sind.

9. Verfahren nach Anspruch 8, **dadurch gekennzeichnet, daß** man für jedes in seinem sequenzspezifischen Kontext nachzuweisende Cytosin mindestens ein zu dem Sequenzkontext komplementäres Oligomer auf der Oberfläche fixiert, welches das zum nachzuweisenden Cytosin komplementäre Guanin enthält und ein weiteres Oligomer, welches an der Stelle des nachzuweisenden Cytosins die Base enthält, welche zu der Base komplementär ist, in welche nicht methylierte Cytosine durch die chemische Behandlung umgewandelt werden.

10. Verfahren nach Anspruch 8 oder 9, **dadurch gekennzeichnet, daß** man für nachzuweisende Cytosin-Positionen solche Oligomere fixiert, die jeweils an die methylierte und unmethylierte Position sowohl auf Plus- als auch auf Minus-Strang spezifisch binden oder/und an die je durch Amplifikation entstehenden komplementären Stränge spezifisch hybridisieren.

11. Verfahren nach einem der Ansprüche 8 bis 10, **dadurch gekennzeichnet, daß** man auf der Oberfläche weitere Oligomere fixiert, welche jeweils an die Sequenz "Sequenzkontext-Thymin-Sequenzkontext" spezifisch binden und/oder welche Cytosin und die durch chemische Behandlung entstandene Base im Plus-Strang, Minus-Strang und den je durch Amplifikation entstehenden komplementären Strängen entstehenden Strängen nachweisen.

12. Verfahren nach den Ansprüchen 8 bis 11, **dadurch gekennzeichnet, daß** man von den Oligomeren an Punkten der Oberfläche(n) Signale detektiert, die für die in der originalen genomischen Probe methylierten, oder unmethylierten oder mutierten spezifisch sind.

13. Verfahren nach Anspruch 12, **dadurch gekennzeichnet, daß** man durch den Vergleich der detektierten Signale der absolute Grad der Methylierung und/oder die Homo- bzw. Heterozygotie ermittelt.

14. Verfahren nach einem der Ansprüche 1 bis 7, **dadurch gekennzeichnet, daß** man die amplifizierten Fragmente der beiden Proben auf je einer Oberfläche fixiert und mit einer nachweisbaren Markierung versehene, sequenzspezifische Oligomere auf diese Oberflächen hybridisiert.

15. Verfahren nach Anspruch 14, daß man die Analyse der hybridisierten sequenzspezifischen Oligomere mittels Massenspektrometrie und bevorzugt einem MALDI Massenspektrometer durchführt.

16. Verfahren nach Anspruch 14, **dadurch gekennzeichnet, daß** man die Analyse der hybridisierten sequenzspezifischen Oligomere mittels Fluoreszenz oder Chemoluminiszenz durchführt.

17. Verfahren nach einem der Ansprüche 1 bis 7 oder 14, **dadurch gekennzeichnet, daß** der Nachweis von Cytosin im Sequenzkontext durch eine Polymerase-Reaktion, welche spezifisch bei Erreichen einer Cytosin-Base im Templat gestoppt wird, und Längenmessung der entstehenden Fragmente erfolgt.

18. Verfahren nach Anspruch 17, **dadurch gekennzeichnet, daß** man die Oligomere, die zum Starten der primer-abhängigen Polymerase-Reaktion benutzt werden, je unterschiedliche Sequenz an unterschiedlichen Orten auf einer Oberfläche fixiert und die Polymerase-Reaktion auf dieser Oberfläche erfolgt.

19. Verfahren nach Anspruch 17 oder 18, **dadurch gekennzeichnet, daß** man die Oligomere, die zum Starten der primer-abhängigen Polymerase-Reaktion benutzt werden, durch eine chemische Reaktion oder durch Licht von der Oberfläche ablöst.

20. Verfahren nach einem der Ansprüche 17 bis 19, **dadurch gekennzeichnet, daß** man zur Termination der Polymerasereaktion an der Position eines Cytosins oder - im Gegenstrang - eines Guanins einen Nukleotidbaustein verwendet, welcher über eine chemische Modifikation auch eine Detektion zum Beispiel durch Fluoreszenz, Chemoluminiszenz oder das Binden eines Antikörpers erlaubt.

21. Verfahren nach Anspruch 17 bis 20, **dadurch gekennzeichnet, daß** man die Detektion der Termination an der Position eines Cytosins oder - im Gegenstrang - eines Guanins über eine Längenmessung der entstehenden Fragmente durch Gelelektorphorese, insbesondere Kapillarelektrophorese durchführt.

22. Verfahren nach einem der Ansprüche 17 bis 19, **dadurch gekennzeichnet, daß** man die Längenmessung der entstehenden Fragmente durch massenspektrometrische Analyse und bevorzugt in einem MALDI Massenspektrometer durchführt.

23. Verfahren nach einem der vorangehenden Ansprüche, **dadurch gekennzeichnet, daß** die Referenz DNA-Sequenz aus einer Datenbank, nämlich aus dem Humangenom-Projekt, stammt.

## Claims

1. A method for distinguishing 5-position methylation changes of cytosine bases and cytosine-to-thymine mutations and for the detection of single nucleotide polymorphisms (SNPs) or point mutations in genomic DNA, **characterized in that**:
a) a genomic DNA sample is treated with sulfite or disulfite in such a way that all of the cytosine bases that are unmethylated at the 5-position of the base are changed, so that a base different in its base-pairing behavior arises, whereas the cytosines methylated at the 5-position remain unchanged and
b) an aliquot of the same genomic DNA sample prior to the chemical treatement according to a) is methylated quantitatively with SSS1 or another methyltransferase and
c) both of the DNA samples treated in this way are investigated for the presence of cytosine by means of the same analytical method and
d) the determined cytosine positions are matched to a reference DNA sequence and
e)it is determined by means of comparing the individual cytosine positions of both samples treated in different ways with the reference sequence whether cytosine is not detectable at a specific position and whether this is due to the fact that cytosine is present in the unmethylated state in the genomic DNA or whether it is present but changed due to a mutation or a polymorphism and thus is not present in the genomic DNA.

2. The method according to one of the preceding claims, further **characterized in that** the two DNA samples or parts of these DNA samples are amplified by a cyclical process, namely polymerase chain reaction or a comparable process prior to the detection of the cytosine base.

3. The method according to claim 2, further **characterized in that** more than 10 different fragments of the treated genomic DNA are produced in one amplification batch.

4. The method according to one of the preceding claims, further **characterized in that** those primers are used for the amplification of the genomic DNA samples, which contain so-called consensus sequences or such sequences important for gene regulation and which thus predominantly bind to regulating or coding sequences.

5. The method according to one of the preceding claims, further **characterized in that** cytosine is detected in the specific context 5'-CpG-3'.

6. The method according to one of the preceding claims, further **characterized in that** the DNA is provided with one or more detectable label(s) in the amplification by incorporation of nucleotide building blocks or oligonucleotides provided with a detectable label.

7. The method according to claim 6, further **characterized in that** the label is detected by fluorescence or chemiluminescence.

8. The method according to one of the preceding claims, further **characterized in that** cytosine is detected by means of hybridizing with oligomers specific for "sequence context-cytosine-sequence context", which [oligomers] are attached in a defined arrangement onto one or more surfaces.

9. The method according to claim 8, further **characterized in that** for each cytosine to be detected in its sequence-specific context, at least one oligomer complementary to the sequence context is attached onto the surface, which contains guanine complementary to the cytosine to be detected, and another oligomer, which contains the base that is complementary to the base to which unmethylated cytosines are converted by the chemical reaction at the site of the cytosine to be detected.

10. The method according to claim 8 or 9, further **characterized in that** for cytosine positions to be detected, those oligomers are attached, which bind specifically each time to the methylated and unmethylated positions both on the plus strand as well as on the minus strand or/and hybridize specifically to the complementary strands that are formed by amplification.

11. The method according to one of claims 8 to 10, further **characterized in that** additional oligomers are attached onto the surface, which [oligomers] bind specifically each time to the sequence "sequence context-thymine-sequence context" and/or which detect cytosine and the base that is formed by chemical treatment in the plus strand, the minus strand and the strands forming by amplification of the compelementary strands that arise.

12. The method according to claims 8 to 11, further **characterized in that** signals that are specific for the methylated or unmethylated or mutated [positions] in the original genomic sample are detected by the oligomers at points of the surface(s).

13. The method according to claim 12, further **characterized in that** the absolute degree of methylation and/or the homozygotic or heterozygotic status is determined by a comparison of the detected signals.

14. The method according to one of claims 1 to 7, further **characterized in that** the amplified fragments of both samples are attached onto a surface and hybridized with sequence-specific oligomers provided with a detectable label on these surfaces.

15. The method according to claim 14, [further **characterized**] in that the analysis of the hybridized sequence-specific oligomers is conducted by means of mass spectrometry and preferably with a MALDI mass spectrometer.

16. The method according to claim 14, further **characterized in that** the analysis of the hybridized sequence-specific oligomers is conducted by means of fluorescence of chemiluminescence.

17. The method according to one of claims 1 to 7 or 14, further **characterized in that** the detection of cytosine is made in the sequence context by a polymerase reaction, which is stopped specifically upon reaching a cytosine base in the template, and the lengths of the fragments that are formed are measured.

18. The method according to claim 17, further **characterized in that** [for] the oligomers that are utilized for initiating the primer-dependant polymerase reaction, each different sequence is fixed at a different site onto a surface and the polymerase reaction is conducted on this surface.

19. The method according to claim 17 or 18, further **characterized in that** the oligomers that are utilized for initiating the primer-dependent polymerase reaction, are stripped from the surface by a chemical reaction or by light.

20. The method according to one of claims 17 to 19, further **characterized in that** for the termination of the polymerase reaction at the position of a cytosine or - in the counterstrand - of a guanine, a nucleotide building block is used, which permits a detection via a chemical modification, for example, by fluorescence, chemiluminescence or the binding of an antibody.

21. The method according to claims 17 to 20, further **characterized in that** the termination at the position of a cytosine or - in the counterstrand - of a guanine is detected by means of a length measurement of the fragments that arise by gel electrophoresis, particularly capillary electrophoresis.

22. The method according to one of claims 17 to 19, further **characterized in that** the length measurement of the fragments that form is conducted by mass-spectrometric analysis and preferably in a MALDI mass spectrometer.

23. The method according to one of the preceding claims, further **characterized in that** the reference DNA sequence originates from a database, namely from the human genome project.

## Revendications

1. Procédé de distinction des modifications de méthylation en position 5 de bases cytosine et de mutations cytosine en thymine et de détection des polymorphismes d'un seul nucléotide (SNP) ou de mutations ponctuelles dans l'ADN génomique, **caractérisé en ce que**
a) on traite un échantillon d'ADN génomique avec le sulfite ou disulfite, de sorte que toutes les bases cytosine non méthylées en position 5 de la base soient modifiées, de sorte que l'on forme un comportement différent d'appariement des bases par des bases différentes alors que les cytosines méthylées en position 5 restent inchangées,
b) un aliquot du même échantillon d'ADN génomique est quantitativement méthylé avant le traitement chimique selon a), avec la SSS1 ou une autre méthyltransférase,
c) les deux échantillons d'ADN ainsi traités sont examinés à l'aide de la même méthode analytique, concernant la présence de cytosines,
d) les positions déterminées des cytosines sont comparées à une séquence d'ADN de référence, et
e) on détermine par la comparaison de chaque position de cytosine dans les deux échantillons traités différemment avec la séquence de référence si en une certaine position, une cytosine n'est pas détectable et si cela repose sur le fait que la cytosine est présente sous forme non méthylée dans l'ADN génomique ou est présente sous forme modifiée par une mutation ou un polymorphisme et ainsi, n'est pas présente dans l'ADN génomique.

2. Procédé selon la revendication précédente, **caractérisé en ce que** l'on amplifie les deux échantillons d'ADN ou des parties de ces échantillons d'ADN avant la détection des bases cytosine, par un processus cyclique, à savoir la réaction en chaîne par polymérase ou un processus comparable.

3. Procédé selon la revendication 2, **caractérisé en ce que** l'on produit dans un lot d'amplification, plus de 10 fragments différents de l'ADN génomique traité.

4. Procédé selon l'une quelconque des revendications précédentes, **caractérisé en ce que** l'on utilise pour l'amplification des échantillons d'ADN génomique, des amorces qui contiennent des séquences dites consensus, importantes pour la régulation génique ou des séquences similaires, et qui se lient ainsi essentiellement aux séquences de régulation ou codantes.

5. Procédé selon l'une quelconque des revendications précédentes, **caractérisé en ce que** l'on détecte les cytosines dans le contexte spécifique 5'-CpG-3'.

6. Procédé selon l'une quelconque des revendications précédentes, **caractérisé en ce que** l'on munit l'ADN d'un ou de plusieurs marqueurs détectables lors de l'amplification, par incorporation d'éléments nucléotidiques ou d'oligonucléotides, munis d'un marqueur détectable.

7. Procédé selon la revendication 6, **caractérisé en ce que** l'on réalise la détection du marquage par fluorescence ou chimioluminescence.

8. Procédé selon l'une quelconque des revendications précédentes, **caractérisé en ce que** l'on détecte les cytosines par hybridation avec des oligomères spécifiques du « contexte de séquence-cytosine-contexte de séquence», lesquels sont fixés selon un ordre défini, sur une ou plusieurs surfaces.

9. Procédé selon la revendication 8, **caractérisé en ce que** l'on fixe sur la surface, pour chaque cytosine à détecter dans son contexte spécifique de séquence, au moins un oligomère complémentaire du contexte de séquence, qui contient une guanine complémentaire à la cytosine à détecter et un autre oligomère, qui contient en la position de la cytosine à détecter, la base qui est complémentaire à la base en laquelle la cytosine non méthylée est transformée par le traitement chimique.

10. Procédé selon la revendication 8 ou 9, **caractérisé en ce que** l'on fixe pour les positions de cytosine à détecter, des oligomères qui se lient spécifiquement, chaque fois en les positions méthylées et non méthylées, non seulement au brin plus, mais également au brin moins et/ou sur lesquels s'hybrident spécifiquement, chaque fois, les brins complémentaires formés par amplification.

11. Procédé selon l'une quelconque des revendications 8 à 10, **caractérisé en ce que** l'on fixe sur la surface, d'autres oligomères, qui se lient spécifiquement à la séquence « contexte de séquence-thymine-contexte de séquence » et/ou qui détectent une cytosine et la base formée par traitement chimique dans le brin plus, le brin moins et les brins formés des brins complémentaires formés par l'amplification.

12. Procédé selon l'une quelconque des revendications 8 à 11, **caractérisé en ce que** l'on détecte parmi les oligomères, en des points de la surface, des signaux qui sont spécifiques de méthylation, non-méthylation ou mutation dans l'échantillon génomique initial.

13. Procédé selon la revendication 12, **caractérisé en ce que** l'on détermine par la comparaison des signaux détectés, le taux absolu de méthylation et/ou d'homo- ou d'hétérozygotie.

14. Procédé selon l'une quelconque des revendications 1 à 7, **caractérisé en ce que** l'on fixe les fragments amplifiés des deux échantillons sur chaque fois une surface et que l'on hybride sur ces surfaces, des oligomères spécifiques de séquence, munis d'un marquage détectable.

15. Procédé selon la revendication 14, **caractérisé en ce que** l'on réalise l'analyse des oligomères spécifiques de séquences hybridés par spectrométrie de masse et de préférence, par un spectromètre de masse MALDI.

16. Procédé selon la revendication 14, **caractérisé en ce que** l'on réalise l'analyse des oligomères spécifiques de séquences hybridés par fluorescence ou chimioluminescence.

17. Procédé selon l'une quelconque des revendications 1 à 7 ou 14, **caractérisé en ce que** l'on réalise la détection de la cytosine dans un contexte de séquence, par une réaction par polymérase, qui s'arrête spécifiquement lorsqu'elle atteint une base cytosine dans la matrice, et mesure de longueur des fragments formés.

18. Procédé selon la revendication 17, **caractérisé en ce que** l'on fixe les oligomères, qui sont utilisés au départ de la réaction par polymérase dépendante d'amorce, en des sites différents de la surface pour des séquences différentes et que l'on réalise la réaction par polymérase sur cette surface.

19. Procédé selon la revendication 17 ou 18, **caractérisé en ce que** l'on élimine de la surface, les oligomères, qui sont utilisés pour le départ de la réaction par polymérase dépendante d'amorce, par une réaction chimique ou par de la lumière.

20. Procédé selon l'une quelconque des revendications 17 à 19, **caractérisé en ce que** l'on utilise, pour la terminaison de la réaction par polymérase en la position d'une cytosine, ou dans le brin opposé, d'une guanine, un élément nucléotidique qui permet également par une modification chimique une détection, par exemple par fluorescence, chimioluminescence ou la liaison d'un anticorps.

21. Procédé selon l'une quelconque des revendications 17 à 20, **caractérisé en ce que** l'on réalise la détection de la terminaison en la position d'une cytosine, ou dans le brin opposé, d'une guanine, par une mesure de longueur des fragments formés par électrophorèse sur gel, en particulier par électrophorèse capillaire.

22. Procédé selon l'une quelconque des revendications 17 à 19, **caractérisé en ce que** l'on réalise la mesure de longueur des fragments formés par analyse par spectrométrie de masse et de préférence, dans un spectromètre de masse MALDI.

23. Procédé selon l'une quelconque des revendications précédentes, **caractérisé en ce que** la séquence de l'ADN de référence provient d'une banque de donnée, à savoir du projet du génome humain.
